# EUROPÄISCHE PATENTSCHRIFT

(11) **EP 1 587 806 B1**
(45) Veröffentlichungstag und Bekanntmachung des Hinweises auf die Patenterteilung: **12.11.2008**
(21) Anmeldenummer: 04700447.8
(22) Anmeldetag: 07.01.2004
(51) Int. Cl.: C07D 487/04, A61K 31/53

(54) **PYRIMIDO 5,4-e 1,2,4 TRIAZIN-5,7-DIONE, VERF AHREN ZU DEREN HERSTELLUNG UND DEREN VERWENDUNG**
PYRIMIDO 5,4-E 1,2,4 TRIAZINE-5,7-DIONES, METHODS FOR PRODUCING THE SAME AND THEIR USE
PYRIMIDO 5,4-E 1,2,4 TRIAZINE-5,7-DIONES, PROCEDES DE FABRICATION DE CES COMPOSES ET UTILISATION DE CEUX-CI

(30) Priorität: 20.01.2003 DE 10301788
(43) Veröffentlichungstag der Anmeldung: 26.10.2005
(73) Patentinhaber: Sanofi-Aventis Deutschland GmbH, 65929 Frankfurt am Main (DE)
(72) Erfinder: PETRY, Stefan, 65926 Frankfurt am Main (DE); BARINGHAUS, Karl-Heinz, 61200 Wölfersheim (DE); TENNAGELS, Norbert, 65926 Frankfurt am Main (DE); MUELLER, Guenter, 65843 Sulzbach (DE)
(86) Internationale Anmeldenummer: PCT/EP2004/000040
(87) Internationale Veröffentlichungsnummer: WO 2004/065387

(56) Entgegenhaltungen:
- WO-A-02/20525
- YONEDA F. ET AL: "Convenient synthesis of oxoflavins, toxoflavin 4-oxides, and 1-demethyltoxoflavins " CHEMICAL AND PHARMACEUTICAL BULLETIN, Bd. 23, Nr. 9, 1975, Seiten 2001-2009, XP009030591
- LIAO T.,K. ET AL: "Synthesis of 1-Demethyltoxoflavin (8-Demethylfervenulin)" JOURNAL OF ORGANIC CHEMISTRY, Bd. 31, 1966, Seiten 900-902, XP002279697
- PATENT ABSTRACTS OF JAPAN vol. 1998, no. 01, 30. Januar 1998 (1998-01-30) & JP 09 255681 A (TAISHO PHARMACEUT CO LTD), 30. September 1997 (1997-09-30) & JP 09 255681 A 30. September 1997 (1997-09-30)

## Beschreibung

Die Erfindung betrifft die Verwendung von Pyrimido[5,4-e][1,2,4]triazin-5,7-dionen sowie deren physiologisch verträgliche Salze.

In JP 96-67814 sind strukturähnliche Verbindungen als Antikrebsmittel beschrieben. In T. Nagamatsu et al., Chem. Pharm. Bull. 1993, 41 (2), 362-8 sind strukturähnliche Verbindungen als Fungizid beschrieben.
In F. Yoneda et al. Tetrahedron Letters, 1971, 851-854 sind strukturähnliche Verbindungen beschrieben.
In JP 09-255681A sind strukturähnliche Verbindungen als Antitumormittel beschrieben.

Der Erfindung lag die Aufgabe zugrunde, Verbindungen zur Verfügung zu stellen, mit denen eine Prävention und Behandlung von Diabetes Typ 1 und 2 möglich ist. Die Verbindungen sollen dazu eine merkliche Senkung des Blutzuckerspiegels bewirken.

Die Erfindung betrifft daher die Verwendung der Verbindungen der Formel I, 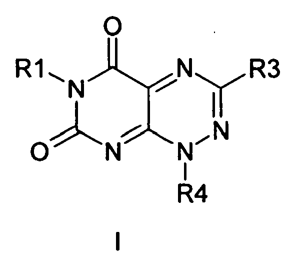 worin bedeuten
- R1: (C₁-C₆)-Alkyl;
- R3: (C₁-C₆)-Alkyl-Phenyl, (C₂-C₆)-Alkenyl-Phenyl, wobei der Phenylring mit F, Cl, Br, OR13 oder R13 substituiert sein kann;
- R4: (C₁-C₆)-Alkyl, (C₁-C₆)-Alkylen-D;
- R13: (C₁-C₆)-Alkyl, Phenyl;
sowie deren physiologisch verträgliche Salze, zur Herstellung eines Medikaments zur Blutzuckersenkung.

Die Erfindung betrifft weiter die Verwendung der Verbindungen der Formel I, 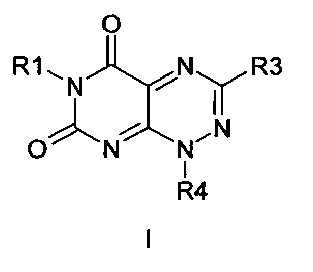 worin bedeuten
- R1: (C₁-C₆)-Alkyl;
- R3: (C₁-C₆)-Alkyl-Phenyl, (C₂-C₆)-Alkenyl-Phenyl, wobei der Phenylring mit F, Cl, Br, OR13 oder R13 substituiert sein kann;
- R4: (C₁-C₆)-Alkyl, (C₁-C₆)-Alkylen-D;
- R13: (C₁-C₆)-Alkyl, Phenyl;
sowie deren physiologisch verträgliche Salze, zur Herstellung eines Medikamentes zur Behandlung des Typ II Diabetes.

Die Erfindung betrifft weiter die Verwendung der Verbindungen der Formel I, 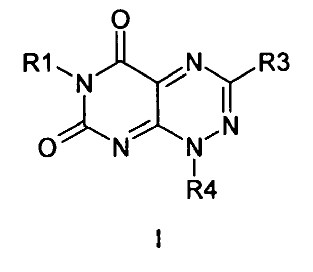 worin bedeuten
- R1: (C₁-C₆)-Alkyl;
- R3: (C₁-C₆)-Alkyl-Phenyl, (C₂-C₆)-Alkenyl-Phenyl, wobei der Phenylring mit F, Cl, Br, OR13 oder R13 substituiert sein kann;
- R4: (C₁-C₆)-Alkyl, (C₁-C₆)-Alkylen-D;
- R13: (C₁-C₆)-Alkyl, Phenyl;
sowie deren physiologisch verträgliche Salze, zur Herstellung eines Medikaments zur Behandlung von Lipid- und Kohlenhydratstoffwechselstörungen.

Die Erfindung betrifft weiter die Verwendung der Verbindungen der Formel I, 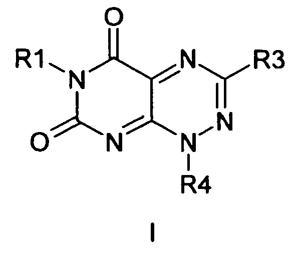 worin bedeuten
- R1: (C₁-C₆)-Alkyl;

- R3: (C₁-C₆)-Alkyl-Phenyl, (C₂-C₆)-Alkenyl-Phenyl, wobei der Phenylring mit F, Cl, Br, OR13 oder R13 substituiert sein kann;
- R4: (C₁-C₆)-Alkyl, (C₁-C₆)-Alkylen-D;
- R13: (C₁-C₆)-Alkyl, Phenyl;
sowie deren physiologisch verträgliche Salze, zur Herstellung eines Medikaments zur Behandlung arteriosklerotischer Erscheinungen.

Die Erfindung betrifft weiter die Verwendung der Verbindungen der Formel I, 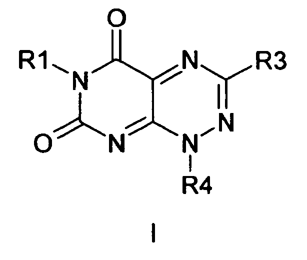 worin bedeuten
- R1: (C₁-C₆)-Alkyl;
- R3: (C₁-C₆)-Alkyl-Phenyl, (C₂-C₆)-Alkenyl-Phenyl, wobei der Phenylring mit F, Cl, Br, OR13 oder R13 substituiert sein kann;
- R4: (C₁-C₆)-Alkyl, (C₁-C₆)-Alkylen-D;
- R13: (C₁-C₆)-Alkyl, Phenyl;
sowie deren physiologisch verträgliche Salze, zur Herstellung eines Medikaments zur Behandlung von Insulin Resistenz.

Die Alkylreste in den Substituenten R1, R3 und R4 können sowohl geradkettig wie verzweigt sein.

Können Reste oder Substituenten mehrfach in den Verbindungen der Formel I auftreten, wie zum Beispiel COOR13, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin, oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel" auf Verbindung(en) der Formel I wie vorstehend beschrieben, sowie ihre Salzeund Solvate wie hierin beschrieben.

Die Verbindung(en) der Formel (I) können auch in Kombination mit weiteren Wirkstoff verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1 % bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Als weitere Wirkstoffe für die Kombinationspräparate sind geeignet:
Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US00/11833, PCT/US00/11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide , BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. Cl-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinyl-methoxy]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten z.B. Naphthalin-1-sulfonsäure-{4-[(4-amino-quinazolin-2-ylamino)-methyl]-cyclohexylmethyl}-amid Hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff Hydrochlorid (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1 H-indol-6-yloxy)-ethylamino]-ethanol Hydrochlorid (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525)), Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1 H-isochinolin-2-carbonsäuretertiärbutylester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

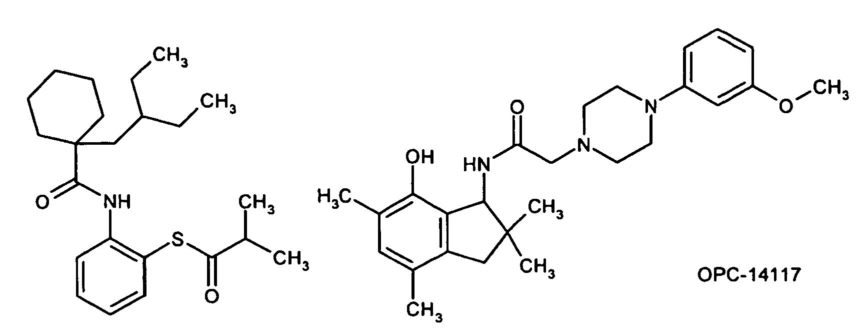

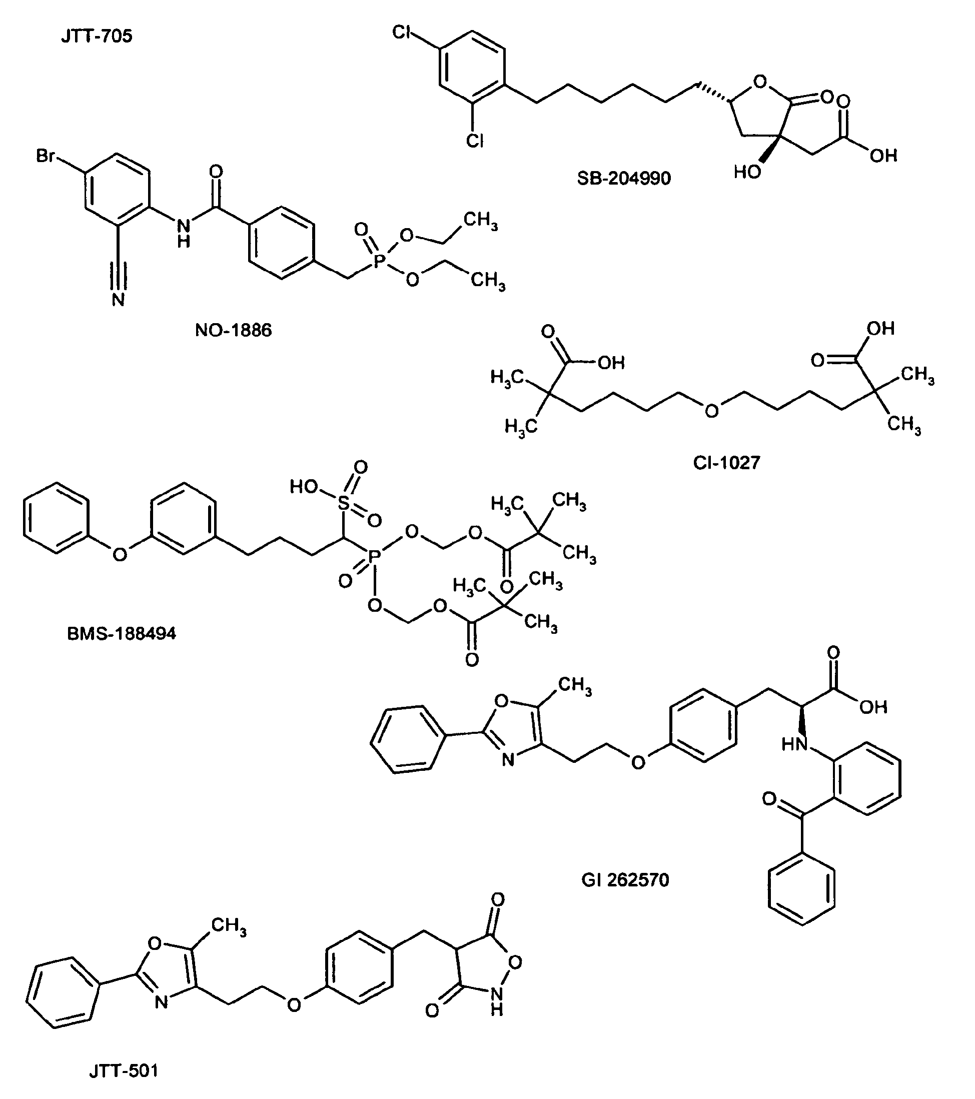

Die nachfolgend aufgeführten Beispiele dienen zur Erläuterung der Erfindung, ohne diese jedoch einzuschränken.

**Tabelle 1: Beispiele der Formel I**

| | | | | |
|---|---|---|---|---|
| 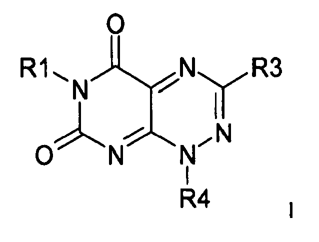 | | | | |

| **Bsp.** | **R1** | **R3** | **R4** | **MS*** |
|---|---|---|---|---|
| **1** | CH₃ | 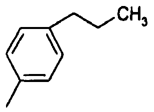 | CH₂-CH₃ | ok |
| **3** | CH₃ | 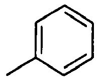 | CH₂-CH₃ | ok |
| **4** | CH₃ | 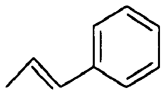 | (CH₂)₂-D | ok |
| **5** | CH₃ | 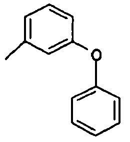 | (CH₂)₃-CH₃ | ok |
| **6** | CH₃ | 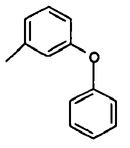 | CH₂-CH₃ | ok |
| **7** | CH₃ | 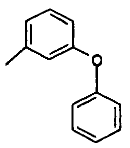 | CH₂-CH₃ | ok |
| **8** | CH₃ | 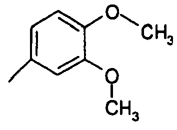 | (CH₂)₃-CH₃ | ok |

| | | | | |
|---|---|---|---|---|
| * Unter der Angabe "MS ist ok" wird verstanden, dass ein Massenspektrum oder HPLC/MS gemessen wurde und in diesem der Molpeak (Molmasse + H⁺) nachgewiesen wurde. | | | | |

Die Verbindungen der Formel I können zur Herstellung von Arzneimitteln verwendet werden.

Solche Arzneimittel eignen sich insbesondere zur Behandlung von Diabetes Typ 1 und 2, Insulinresistenz und krankhafter Dickleibigkeit. Sie eignen sich darüber hinaus auch zur Behandlung von überhöhten Blutfettwerten, Bluthochdruck, Atherosklerose, Fehlfunktionen des Immunsystems, Autoimmunkrankheiten, allergischen Krankheiten wie Asthma, bei Osteoporose, Proliferationsstörungen wie Krebs und Psoriasis, Krankheiten mit verminderter oder erhöhter Produktion von Wachstumsfaktoren, Hormonen oder Cytokinen, die die Freisetzung von Wachstumshormonen auslösen, Infektionskrankheiten oder Erkrankungen des Nervensystems wie Alzheimer und Schizophrenie.

Die Verbindungen der Formel I können weiterhin zur Herstellung eines Arzneimittels verwendet werden, wobei dieses Arzneimittel eine PTPase inhibiert. Als PTPasen können dabei insbesondere PTP1B, CD45, LAR, SHP-1, SHP-2, PTPa oder HePTP auftreten.

Schließlich können Verbindungen der Formel I zur Herstellung eines Arzneimittels verwendet werden, wobei dieses Arzneimittel zur Behandlung von Krankheiten insbesondere Diabetes Typ 1 und 2, Insulinresistenz, krankhafter Dickleibigkeit, überhöhten Blutfettwerten, Bluthochdruck, Atherosklerose, Fehlfunktionen des Immunsystems, Autoimmunkrankheiten, allergischen Krankheiten wie Asthma, bei Osteoporose, Proliferationsstörungen wie Krebs und Psoriasis, Krankheiten mit verminderter oder erhöhter Produktion von Wachstumsfaktoren, Hormonen oder Cytokinen, die die Freisetzung von Wachstumshormonen auslösen, Erkrankungen des Nervensystems wie Alzheimer und Schizophrenie und Infektionskrankheiten eingesetzt werden kann.

Weiterhin können Verbindungen der Formel I pur Herstellung eines Arzneimittels zur Behandlung von Diabetischen Spätschäden, (wie z.B. Nephropatie, Retinopathie, Neuropathie) sowie Herzinfarkt, Myocardialem Infarkt, peripheren arteriellen Verschlusskrankheiten, Thrombosen, Arteriosklerose, Syndrom X, Obesitas, Insulinresistenz, Entzündungen, Immunkrankheiten, Autoimmunkrankheiten, wie z.B. AIDS, Asthma, Osteoporose, Krebs, Psoriasis, Alzheimer, Schizophrenie und Infektionskrankheiten eingesetzt werden.

Die Erfindung betrifft die Herstellung eines Arzneimittels enthaltend wenigstens eine Verbindung dieser Erfindung, wobei der Wirkstoff mit einem pharmazeutisch geeigneten Träger vermischt wird und diese Mischung in eine für die Verabreichung geeignete Form gebracht wird.

### Die Wirksamkeit der Verbindungen wurde wie folgt getestet:

### Hemmung der Phosphotyrosinphosphatase 1B (PTP1B)

Im folgenden wird Aufbau und Durchführung eines in vitro Assays zum Nachweis einer Phosphatase inhibierenden Wirkung der erfindungsgemäßen Verbindungen dargestellt. Beschrieben wird die Gewinnung der Enzympräparation und die Durchführung des Assays.

Gewinnung der Enzympräparation:
A) Zellkultur:
   Sf9 (invitrogen) Zellen werden in Spinnerflaschen bei 28°C in Grace's supplementiertem Medium (Gibco-BRL) mit 10% Hitze-inaktiviertem fetalen Kälberserum (Gibco-BRL) following the protocol of Summers and Smith (A Manual for Methods for Baculoviruns Vectors and Insect Culture Procedures [Bulletin No. 15555]. Texas A & M University, Texas Agricultural Experiment Station, College Station, TX, 1987) kultiviert.
   Konstruktion von rekombinanten Baculovirus Transfervektoren: cDNA kodierend für die regulatorischen and katalytischen Domänen der menschlichen PTP1 B, aber ohne die carboxy-terminale hydrophobe Region (entsprechend 1-299 aa) wurde über Polymerasekettenreaktion über Primer mit günstigen Klonierungsstellen und geeigneten cDNA Matrizen erhalten und dann in Baculovirusexpressionvektoren (Amersham Pharmacia Biotech.) kloniert. Die rekombinanten Baculoviren wurden mit Hilfe des Bac-to-Bac Baculovirus Expressionsystems (Gibco-BRL) hergestellt. In Kürze, das Gen wurde in das pFASTBAC Donorplasmid kloniert, welches am 5'-Ende der cDNA eine FLAG Sequenz besitzt. Das resultierende Plasmid wurde in kompetente DH10BAC *Escherichia coli* Zellen transformiert. Nach der Transposition und Antibiotikaselektion wurde die rekombinante Plasmid-DNA von selektierten *E*. *coli* Kolonien isoliert und dann für die Transfektion von Sf9 Insektenzellen benutzt. Der Viruspartikel im Überstandsmedium wurde dreimal amplifiziert bis auf ein virales Stockvolumen von 500 ml.
B) Produktion of rekombinantem Protein:
   Baculovirusinfection einer 500-ml Spinnerkultur von Sf9 Zellen wurde im wesentlichen durchgeführt wie von Summers und Smith beschrieben (s.o.). Sf9 Zellen bei einer Dichte von 1-3 x 10⁶ Zellen/ml wurden durch Zentrifugation bei 300 g für 5 min pelletiert, der Überstand wurde entfernt und die Zellen in einer Dichte von 1 x 10⁷ Zellen/ml in einem geeigneten rekombinanten Viralstock (MOI 10) resuspendiert. Nach sachtem Schütteln für 1.5 Std. bei Raumtemperatur wurde frisches Medium hinzugegeben, um eine Zelldichte von 1 x 10⁶ Zellen/ml zu erreichen. Die Zellen wurden dann in Suspension bei 28°C für geeignete Perioden nach Postinfektion kultiviert.
C) Zelluläre Fraktionierung und Gesamtzellextrakte von infizierten Sf9 Zellen:
   Eine geeignete Zeit nach der Postinfektion wurden Aliquots einer Analyse der Proteinexpression durch SDS-PAGE und Westernblotanalyse unterzogen. Die zelluläre Fraktionierung wurde durchgeführt wie beschrieben (Cromlish, W. and Kennedy, B. Biochem. Pharmacol. 52: 1777-1785, 1996). Gesamtzellextrakte wurden von 1-ml Aliquots der infizierten Sf9 Zellen nach bestimmten Zeiten Postinfektion gewonnen. Die pelletierten Zellen (300 *g*, 5 min) wurden einmal in Phosphategepufferter Saline (4°C) gewaschen, resuspendiert in 50 µl Wasser und durch wiederholtes Einfrieren/Auftauen aufgeschlossen. Proteinkonzentrationen wurden mit Hilfe der Bradfordmethode (Pierce) und Rinderserumalbumin als Standard bestimmt.

Durchführung des Assays:
A) Dephosphorylierung eines Phosphopeptids:
   Dieser Assay beruht auf der Freisetzung von Phosphat aus einem Konsensussubstratpeptid, welches im nanomolaren Konzentrationsbereich durch die Malachitgrün-Ammoniummolybdate-Methode (Lanzetta, P.A., Alvarez, L.J., Reinach, P.S., Candia, O.A. Anal Biochem. 100: 95-97, 1979) adaptiert für das Mikrotiterplattenformat nachgewiesen wird. Das Dodecatrisphosphopeptid, TRDIYETDYYRK (Biotrend, Köln) entpricht den Aminosäuren 1142-1153 der katalytischen Domaäne des Insulinrezeptors und wird (auto)phosphoryliert an den Tyrosinresten 1146, 1150, und 1151. Die rekombinante hPTP1B wurde mit Assaypuffer verdünnt (40 mM Tris/HCl, pH 7.4, 1 mM EDTA, 20 mM DTT), entsprechend einer Aktivität von 1000-1500 nmol/min/mg Protein und (eine 20 µl-Portion) dann vorinkubiert (15 min, 30°C) in Ab- oder Anwesenheit der Testsubstanz (5 µl) in der gewünschten Konzentration (Endkonz. DMSO 2 % max.) in einem Gesamtvolumen von 90 µl (Assaypuffer). Zum Start der Dephosphorylierungsreaktion wurde das Peptidsubstrat (10 µl, prewarmed at 30°C) zur vorinkubierten Enzympräparation mit oder ohne Testsubstanz (Endkonz. 0.2-200 µM) hinzugegeben und die Inkubation für 1 Std. fortgesetzt. Die Reaktion wurde beendet durch Hinzufügen von 100 µl Malachitgrünhydrochlorid (0.45 %, 3 Teile), Ammoniummolybdattetrahydrat (4.2 % in 4 N HCl, 1 Teil) und 0.5 % Tween 20 als Stoplösung. Nach 30 min Inkubation bei 22°C für die Entwicklung der Farbe wurde die Absorption bei 650 nm mit Hilfe eines Mikrotiterplattenlesegeräts (Molecular Devices) bestimmt. Proben und Leerwerte wurden als Dreifachwerte gemessen. Die PTP1B Aktivität wurde als Nanomole an freigesetztem Phosphat pro min und mg Protein mit Kaliumphosphat als Standard berechnet. Die Inhibition der rekombinanten hPTP1B durch Testsubstanzen wurde als Prozent der Phosphatasekontrolle berechnet. Die IC₅₀-Werte zeigen signifikante Übereinstimmung mit einer Vier-Parameter-nichtlinearen logistischen Regressionskurve.
B) Spaltung von p-Nitrophenylphosphat:
   Dieser Assay beruht auf der Absorptionsveränderung des nicht-physiologischen Substrats *p*-Nitrophenylphosphat während der Spaltung zu Nitrophenol unter Standardbedingungen (Tonks, N.K., Diltz, C.D:, Fischer, E.H. J. Biol. Chem. 263: 6731-6737, 1988; Burke T.R., Ye, B., Yan, X.J., Wang, S.M., Jia, Z.C., Chen, L., Zhang, Z.Y., Barford, D. Biochemistry 35: 15989-15996, 1996). Die Inhibitoren werden in geeigneter Verdünnung zu den Reaktionsgemischen pipettiert, die 0.5-5 mM *p*-Nitrophenylphosphat enthalten. Die folgenden Puffer wurden benutzt (Gesamtvolumen 100 µl): (a) 100 mM Natriumazetat (pH 5.5), 50 mM NaCl, 0.1 % (w/v) Rinderserumalbumin, 5 mM Glutathion, 5 mM DTT, 0.4 mM EGTA und 1 mM EDTA; (b) 50 mM Hepes/KOH (pH 7.4), 100 mM NaCl, 0.1 % (w/v) Rinderserumalbumin, 5 mM Glutathion, 5 mM DTT und 1 mM EDTA. Die Reaktion wurde gestartet durch Zugabe von Enzym und in Mikrotiterplatten bei 25°C für 1 Std. durchgeführt. Die Reaktion wurde beendet durch Zugabe 100 µl 0.2 N NaOH. Die Enzymaktivität wurde bestimmt durch Messung der Absorption bei 405 nm mit geeigneten Korrekturen für Absorption der Testsubstanzen und von *p-*Nitrophenylphosphat. Die Ergebnisse wurden als Prozent der Kontrolle ausgedrückt, in dem die Menge an gebildetem *p*-Nitrophenol in den Testsubstanz-behandelten Proben (nmol/min/mg Protein) mit der Menge in den unbehandelten Proben verglichen wurde. Der Mittelwert und die Standardabweichung wurden berechnet, die IC50-Werte wurden durch Regressionsanalyse des linearen Anteils der Hemmkurven bestimmt.
C) Spaltung von DIFMUP
   Dieser Assay beruht auf der Absorptionsveränderung des nicht-physiologischen Substrats 6,8-Difluoro-4-methylumbelliferylphosphat (DFMUP) während der Spaltung zu 6,8-Difluoro-4-methylumbelliferyl (interne Nr. DEAV2002/0001 DE NP).
   Die Reaktion erfolgt in einer schwarzen Mikrotiterplatte bei einer Temperatur von 37°C. Es werden 120 µl Reaktionspuffer bereitgestellt, der die folgenden Komponenten enthält: 100 ng/ ml rekombinante humane Proteintyrosinphosphatase PTP1b; 50 mM Hepes pH 6,9; 150 mM NaCl; 1 mM EDTA;2 mM DTT und Inhibitoren in geeigneter Verdünnung. Die Phosphatasereaktion wird durch Zugabe von 15 µl DIFMUP-Lösung gestartet, der das Substrat mit der 10fachen Konzentrationen der gewünschten Endkonzentration im Endvolumen enthält) und die Fluoreszenz bei in einem Fluoreszenz-Mikrotiterplatten-Photometer bei 358-455 nm in Zeitintervallen von 30 Sekunden über 15 Minuten gemessen. Maß für die Enzymaktivität ist die gesteigerte Fluoreszenz, welche grafisch dargestellt werden kann. In Abhängigkeit der verwendeten Inhibitorkonzentration ergibt sich eine Reduktion der enzymatischen Aktivität, die Inhibitiorkonzentration, bei welcher eine halbmaximale Enzymaktivität beobachtet wird, bezeichnet man als IC50.

**Tabelle 2: Biologische Aktivität**

| Bsp. gemäß Tabellen 1 | IC50 µM |
|---|---|
| 1 | 0,75 |
| 2 | 0,38 |
| 3 | 0,24 |
| 4 | 0,22 |
| 5 | 5,2 |
| 6 | 0,44 |
| 7 | 0,7 |
| 8 | 2,5 |

Aus der Tabelle ist abzulesen, dass die Verbindungen der Formel I die Aktivität der Phosphotyrosin 1 B hemmen. Deshalb sind sie zur Senkung des Blutzucker- und Insulinspiegels gut geeignet sowie zur Prävention und Behandlung von Diabetes Typ 1 und Typ 2.

Nachfolgend wird die Herstellung einiger Beispiele detailliert beschrieben, die übrigen Verbindungen der Formel I wurden analog erhalten:

### Experimenteller Teil:

### Chloruracil (1)

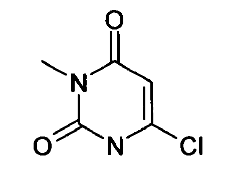

Barbitursäure (10 g, 78,1 mmol) wird bei 0° C vorsichtig mit Phosphorylchlorid (55 mL) versetzt. Anschließend tropft man Wasser zu (1,7 mL), so dass die Temperatur 5° C nicht übersteigt. Der Ansatz wird 5 h unter Rückfluß gekocht und nach Abkühlen auf Eis gegossen. Das Produkt wird mit Ethylacetat (3x100 mL) extrahiert und getrocknet (Na₂ SO₄). Der Ansatz wird filtriert und das Lösungsmittel im Vakuum abdestilliert

Ausbeute: 10,7 g (93%)

### Ethylhydrazin (2)

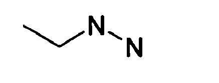

Zu Hydrazinhydrat (250 mL) tropft man unter intensivem Rühren eine Lösung von Ethylbromid (38 mL) in Ethanol (50 mL) zu, so dass die Temperatur 30° C nicht übersteigt. Nach beendeter Zugabe wird noch 2 h gerührt. Der Ansatz wird mit Bariumoxid versetzt und das Produkt über eine Vigreuxkolonne destilliert.

Ausbeute: 37 mL.

### 6-(N-Ethylhydrazino)-3-methyl-1H-pyrimidin-2,4-dion (3)

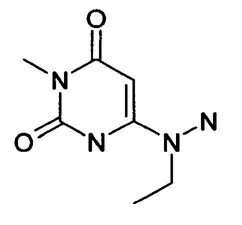

Verbindung 1 (1,5 g , 8 mmol) wird mit Ethylhydrazin (2 mL) versetzt und der Ansatz bein 60 °C gerührt. Nach 3,5 h war dünnschichtchromatogrtaphisch kein Auwsgangsmaterial mehr nachweisbar. Der Ansatz wird im Vakuum eingeengt und das Produkt durch präparative HPLC gereinigt.

Ausbeute: 582 mg

### Beispiel 1 und 2:

### 1-Ethyl-6-methyl-3-phenyl-1H-pyrimido[5,4-e][1,2,4]triazin-5,7-dion (4) und 1-Ethyl-6-methyl-4-oxy-3-phenyl-1H-pyrimido[5,4-e][1,2,4]triazin-5,7-dion (5).

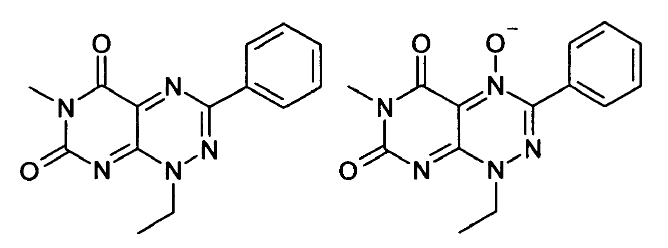

Zu einer Lösung von Verbindung **3** (115 mg, 0.54 mmol) in Eisessig (2 mL) wird Benzaldehyd (55 µL, 0.54 mmol) zugegeben und der Ansatz 20 min bei 10° C gerührt. Anschließend versetzt man mit wässriger Natriumnitritlösung (40 mg NaNO2 in 100 µL Wasser) und rührt noch 30 min bei 10° C. Der Ansatz wird auf Eiswasser gegossen und das Produkt mit Ethylacetat (3x30 mL) extrahiert. Die organischen Phasen werden getrocknet (Na2SO4) und das Lösungsmittel im Vakuum abdestilliert. Der Rückstand wird durch Flash Chromatographie (7:3, Toluol-Ethylacetat) gereinigt. Man erhält 1-Ethyl-6-methyl-3-phenyl-1 H-pyrimido[5,4-e][1,2,4]triazin-5,7-dion (44 mg, 28,8 %) und 1-Ethyl-4-oxy-3-phenyl-6-propyl-1 H-pyrimido[5,4-e][1,2,4]triazin-5,7-dion (41 mg, 32 %)

1-Ethyl-6-methyl-3-phenyl-1 H-pyrimido[5,4-e][1,2,4]triazin-5,7-dion

¹H-NMR: δ = 7,75 (dd, 2 H, aryl), 7,6 (m, 3 H, aryl), 4,4 (q, 2 H, CH₂); 3,3 (3 H, NCH₃), 1,4 (t, 3 H, CH₃)
MS (M+1): 284,2

1-Ethyl-6-methyl-4-oxy-3-phenyl-1H-pyrimido[5,4-e][1,2,4]triazin-5,7-dion

¹H-NMR: δ = 8.2 (dd, 2 H, aryl), 7,6 (m, 3 H, aryl), 4,5 (q, 2 H, CH₂); 3,3 (3 H, NCH₃), 1,5 (t, 3 H, CH₃)
MS (M+1): 300.

### Beispiel 3 und 4:

### 1-Ethyl-3,6-dimethyl-1H-pyrimido[5,4-e][1,2,4]triazin-5,7-dion (6) und 1-Ethyl-3,6-dimethyl-4-oxy-1H-pyrimido[5,4-e][1,2,4]triazin-5,7-dion (7).

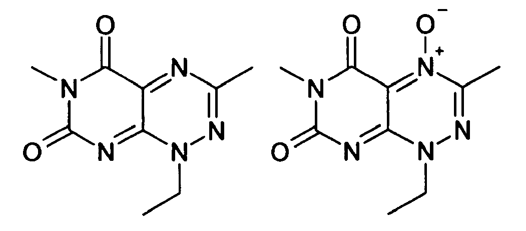

Die Verbindungen **6** und **7** werden wie für **4** und **5** beschrieben durch Umsetzen des Hydrazins **3** (184 mg, 1 mmol) mit Acetaldehyd (57 µL, 1 mmol) hergestellt.

1-Ethyl-3,6-dimethyl-1 H-pyrimido[5,4-e][1,2,4]triazin-5,7-dion (6)
Ausbeute: 62 mg (28%)
¹H-NMR: δ = 4,4 (q, 2 H, NCH₂CH₃); 3,5 (s, 3 H, CH₃), 3,4 (s, 3 H, NCH₃); 1,45 (t, 3H, NCH₂CH₃).

MS (221,22): 222. (M+H).

1-Ethyl-3,6-dimethyl-4-oxy-1H-pyrimido[5,4-e][1,2,4]triazin-5,7-dion (7).
Ausbeute: 21 mg (9%)
¹H-NMR: δ = 4,57 (q, 2 H, CH₂), 3,5 (s, 3 H, CH₃), 1,5 (t, 3 H, CH₃)
MS (237,22): 238,3. (M+H).

### Beispiel 5:

### 1-Ethyl-6-methyl-3-(4-propylphenyl)-1H-pyrimido[5,4-e][1,2,4]triazin-5,7-dion (8)

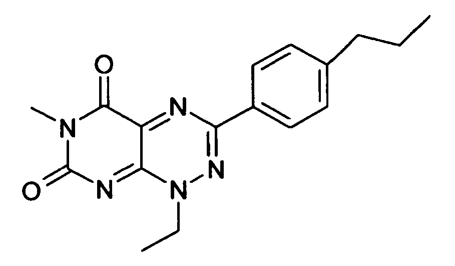

Verbindung **8** wurde wie für Verbindung **4** und **5** beschrieben durch Umsetzung des Hydrazins **3** (100 mg, 0,54 mmol) mit 4-Propylbenzaldehyd (80 mg, 0.54 mmol)) erhalten und durch Flash Chromatographie gereinigt
Ausbeute: 43 mg (24,5%)

¹H-NMR: δ = 8,15 (d, 2 H, aryl), 7,4 (d, 2 H, aryl), 4,5 (q, 2 H, NCH₂CH3); 3,35 (s, 3 H, NCH₃) 2,65 (t, 2 H, benzyl. CH₂), 1,65 (m, 2 H, CH₂CH₂CH₃), 1,45 (t, 3 H, NCH₂CH₃), 0,95 (t, 3H, CH₂CH₂CH₃).
MS (325,37): 326,16 (M+H).

### Beispiel 6 und 7:

### 1-Ethyl-6-methyl-3-phenyl-1H-pyrimido[5,4-e][1,2,4]triazin-5,7-dion (9) und 1-Ethyl-6-methyl-4-oxy-3-phenyl-1H-pyrimido[5,4-e][1,2,4]triazin-5,7-dion (10)

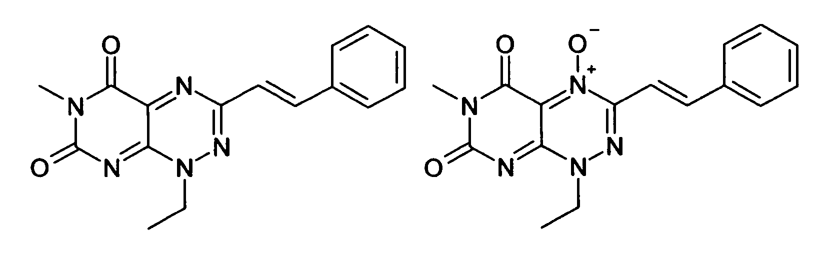

Die Verbindungen **9** und **10** wurden wie für Verbindung **4** und **5** beschrieben durch Umsetzung des Hydrazins **3** (100 mg, 0,54 mmol) mit Zimtaldehyd (71 mg, 0.54 mmol) erhalten und durch Flash Chromatographie gereinigt.

1-Ethyl-6-methyl-3-phenyl-1 H-pyrimido[5,4-e][1,2,4]triazin-5,7-dion (9)

Ausbeute: 52 mg (31%)

¹H-NMR: δ = 7,9 (m, 3 H, 2 aryl-H, CH=CH), 7,4 (m, 3 H, aryl H); 7,3 (d, 1 H CH=CH), 4,4 (q, 2 H, NCH₂CH3); 3,3 (s, 3 H, NCH₃); 1,4 (t, 3 H, NCH₂CH₃).
MS (309,33): 310 (M+H).

Ethyl-6-methyl-4-oxy-3-phenyl-1H-pyrimido[5,4-e][1,2,4]triazin-5,7-dion (10)

Ausbeute: 36 mg (20,5 %)

¹H-NMR: δ = 7,75 (m, 3 H, 2 aryl-H, CH=CH), 7,45 (m, 3 H, 2 aryl H, CH=CH), 4,4 (q, 2 H, NCH₂CH3); 3,3 (s, 3 H, NCH₃); 1,4 (t, 3 H, NCH₂CH₃).
MS (TOF MS ES+; 325,33): 325.

### Beispiel 8:

### 1-Ethyl-6-methyl-3-(3-phenoxyphenyl)-1H-pyrimido[5,4-e][1,2,4]triazin-5,7-dion (11)

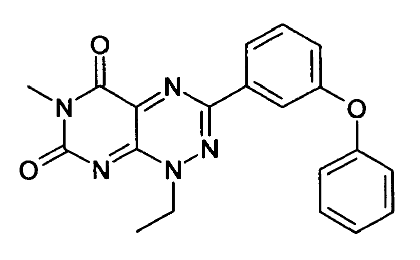

Die Verbindung 11 wurde wie für Verbindung 4 beschrieben durch Umsetzung des Hydrazins 3 (100 mg, 0,54 mmol) mit 3-Phenoxybenzaldehyd (94 µL, 0,54 mmol) erhalten und durch Flash Chromatographie (2:1 Toluol-EtOAc) gereinigt.

Ausbeute: 88 mg (43%)

¹H-NMR: δ = 8,05-7 (m, 9 H, aryl-H); 4,5 (q, 2 H, NCH₂CH3); 3,3 (s, 3 H, NCH₃); 1,45 (t, 3 H, NCH₂CH₃).MS (TOF MS ES+; 375,39): 375.

### 1-Propylbarbitursäure (12)

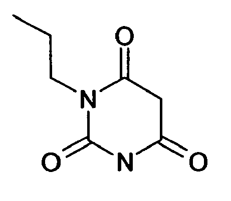

Ethanol (60 mL) wird mit metallischem Natrium (2,6 g, 113 mmol) versetzt. Der Ansatz wird gerührt, bis sich das Natrium vollstaändig abreagiert hat. Anschließend gibt man Malonsäurediethylester (116 mL, 105 mmol) und n-Propylharnstoff (10 g, 98 mmopl) zu und rührt 5h unter Rückfluß. Nach Zugabe von conc. HCl (5 mL) und heißem Wasser (45 mL) wird filtriert und das Filtrat im Vakuum eingeengt. Der Rückstand wird mit Ethanol versetzt und gerührt. Der Feststoff wird abgesaugt und getrocknet.
Ausbeute: 10,68 g (64%).

NMR: 726434 (32165-91)

### 6-Chloro-3-propyl-1 H-pyrimidine-2,4-dione (13)

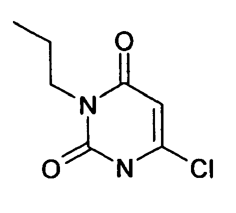

Die Verbindung **13** wurde wie für Verbindung **1** beschrieben durch Umsetzung der Barbitursäure **12** (10 g, 58,8 mmol) mit POCl3 (55 mL) erhalten.

Ausbeute: 2,6 g (23,5%)

### 6-(N-Ethylhydrazino)-3-propyl-1 H-pyrimidin-2,4-dion (14)

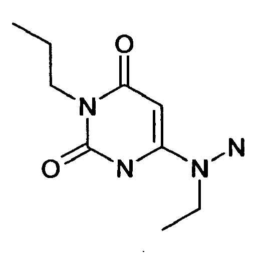

Verbindung **14** wird wie für **3** beschrieben durch Umsetzung von Verbindung **13** (1,5 g, 8 mmol) mit Ethylhydrazin (2 mL) erhalten.

Ausbeute: 528 mg (30,4 %)

### Beispiel 9 und 10:

### 1-Ethyl-6-propyl-3-phenyl-1H-pyrimido[5,4-e][1,2,4]triazin-5,7-dion (15) und 1-Ethyl-6-propyl-4-oxy-3-phenyl-1H-pyrimido[5,4-e][1,2,4]triazin-5,7-dion (16)

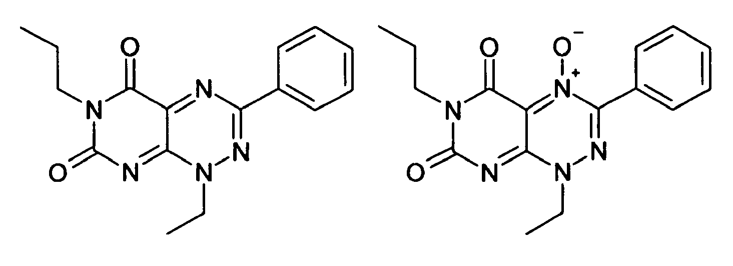

Die Verbindungen **15** und **16** werden wie für **4** und **5** beschrieben durch Umsetzen von Verbindung **14** (115 mg, 0,54 mmol) mit Benzaldehyd (55 µL, 0,54 mmol) hergestellt.

1-Ethyl-6-propyl-3-phenyl-1H-pyrimido[5,4-e][1,2,4]triazin-5,7-dion (15)

Ausbeute: 44 mg (26 %)
¹H-NMR: δ = 8,2 (m, 2 H, aryl-H), 7,6 (m, 3 H, aryl-H); 4,5 (q, 2 H, NCH₂CH3); 3,85 (q, 2 H, NCH₂CH₂CH₃); 1,6 (dq, 2 H, NCH₂CH₂CH₃); 1, 5 (t, 3 H, NCH₂CH₃); 0,9 (t, 3 H, NCH₂CH₂CH₃).

1-Ethyl-6-propyl-4-oxy-3-phenyl-1H-pyrimido[5,4-e][1,2,4]triazin-5,7-dion (16)

Ausbeute: 41 mg (23,2 %)
¹H-NMR: δ = 8,0 (m, 2 H, aryl-H), 7,6 (m, 3 H, aryl-H); 4,4 (q, 2 H, NCH₂CH3), 3,85 (q, 2 H, NCH₂CH₂CH₃); 1,58 (dq, 2 H, NCH₂CH₂CH₃); 1, 4 (t, 3 H, NCH₂CH₃); 0.9 (t, 3 H, NCH₂CH₂CH₃).

### 1-Ethyl-6-propyl-3-[3,4-dimethoxyphenyl]-1H-pyrimido[5,4-e][1,2,4]triazin-5,7-dion (17)

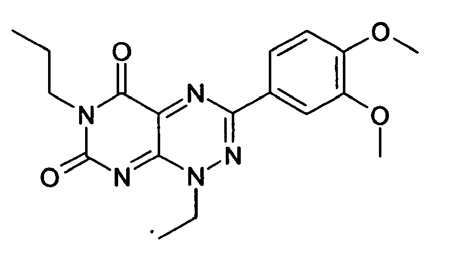

Verbindung **17** wird wie für **4** beschrieben durch Umsetzen des Hydrazins **13** (115 mg, 0,54 mmol) mit 3,4-Dimethoxybenzaldehyd (90 mg, 0,54 mmol) hergestellt.

Ausbeute: 31 mg (15,4 %).
¹H-NMR: δ = 7,85 (dd, 1 H, aryl-H); 7,7 (d, 1 H, aryl-H); 7, (dd, 1 H, aryl-H); 4,5 (q, 2 H, NCH₂CH3); 3,85 (m, 8 H, 2xOCH₃, NCH₂CH₂CH₃); 1,6 (dt, 2 H, NCH₂CH₂CH₃); 1, 45 (t, 3 H, NCH₂CH₃); 0.9 (t, 3 H, NCH₂CH₂CH₃).

### 1-Ethyl-6-propyl-3-[3-phenoxyphenyl]-1H-pyrimido[5,4-e][1,2,4]triazin-5,7-dion (18) und

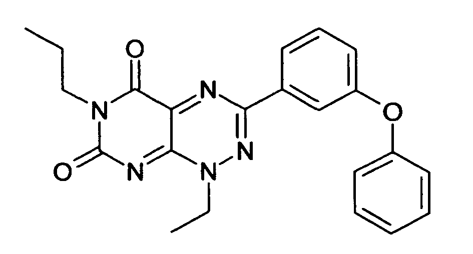

Verbindung **18** wird wie für **4** beschrieben durch Umsetzen des Hydrazins 3 (115 mg, 0,54 mmol) mit 3-Phenoxybenzaldehyd (93 µL, 0,54 mmol) hergestellt.

Ausbeute: 78 mg (35,8 %)
¹H-NMR: δ = 7,95 (dd, 1 H, aryl-H); 7,8 (d, 1 H, aryl-H); 7,63, (dd, 1 H, aryl-H); 7,43 (m, 2 H, aryl-H); 7,2 (m, 2 H, aryl-H); 7,1 (m, 2 H, aryl-H); 4,45 (q, 2 H, NCH₂CH₃); 3,85 (dt, 2 H, NCH₂CH₂CH₃); 1,6 (m, 2 H, NCH₂CH₂CH₃); 1, 45 (t, 3 H, NCH₂CH₃); 0.9 (t, 3 H, NCH₂CH₂CH₃).

### 6-(N-Butylhydrazino)-3-metyl-1H-pyrimidin-2,4-dion (19)

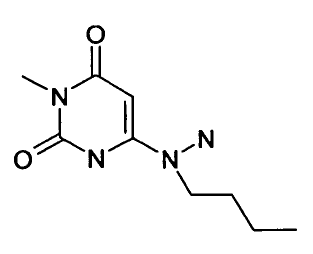

Verbindung 19 wird wie für 3 beschrieben durch Umsetzen von Chloruracil 1 (1 g, 6,2 mmol) mit Butylhydrazin (5,3 g, 60 mmol) hergestellt.
Ausbeute: 1,1 g (83%)

### 1-Butyl-6-metyl-3-[3-phenoxypheny)]-1H-pyrimido[5,4-e][1,2,4]triazin-5,7-dion (20)

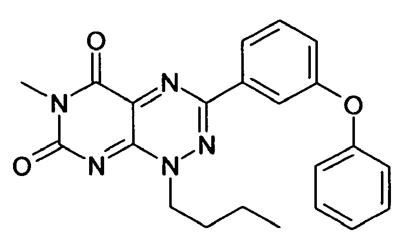

Verbindung 20 wird wie für 4 beschrieben durch Umsetzen des Hydrazins 19 (176 mg, 0,54 mmol) mit 3-Phenoxybenzaldehyd (94 µL, 0,54 mmol) hergestellt.

Ausbeute: 75 mg (34,5%)
¹H-NMR: δ = 8,05 (m, 1 H, aryl-H); 7,8 (d, 1 H, aryl-H); 7,75, (dd, 1 H, aryl-H); 7,43 (m, 2 H, aryl-H); 7,15 (m, 2 H, aryl-H); 7,1 (m, 2 H, aryl-H); 3,9 (q, 2 H, NCH₂CH₂CH₂CH₃); 3,1 (s, 3 H, NCH₃); 1,6-1,3 (m, 4 H, NCH₂CH₂CH₂CH₃); 0.9 (q, 2 H, NCH₂CH₂CH₂CH₃);

### 1-Butyl-6-metyl-3-[3,4-diemthoxyphenyl]-1H-pyrimido[5,4-e][1,2,4]triazin-5, 7-dion (21)

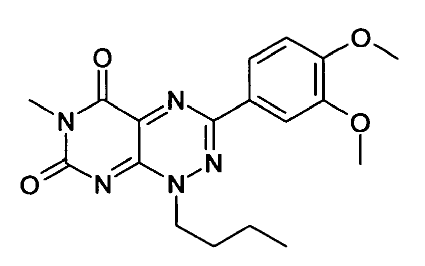

Verbindung **21** wird wie für **4** beschrieben durch Umsetzen des Hydrazins 20 (176 mg, 0,54 mmol) mit 3,4-Dimethoxybenzaldehyd (88 mg, 0,54 mmol) hergestellt. Ausbeute: 24 mg (12%)

¹H-NMR: δ = 7,95 (s, 1 H, aryl-H); 7,6 (d, 1 H, aryl-H); 7,45, (dd, 1 H, aryl-H); 3,9-3,8 (m, 8 H, 2xOCH₃, NCH₂CH₂CH₂CH₃); 3,1 (s, 3 H, NCH₃); 1,6-1,2 (m, 4 H, NCH₂CH₂CH₂CH₃); 0.9 (q, 2 H, NCH₂CH₂CH₂CH₃);

### 1-Butyl-6-metyl-3-phenyl-1H-pyrimido[5,4-e][1,2,4]triazin-5,7-dion (22)

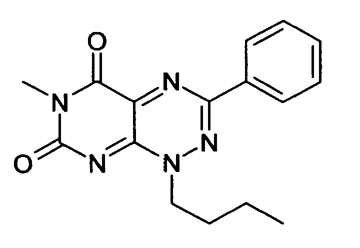

Verbindung 22 wird wie für 4 beschrieben durch Umsetzen des Hydrazins 20 (176 mg, 0,54 mmol) mit Benzaldehyd (55 µL, 0,54 mmol) hergestellt.

Ausbeute: 13 mg (7,8 %)

## Patentansprüche

1. Verwendung der Verbindungen der Formel I, 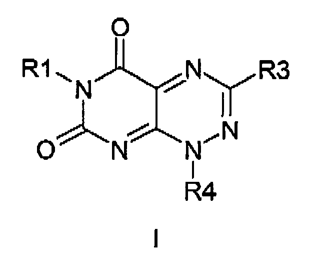 worin bedeuten
R1 (C₁-C₆)-Alkyl;
R3 (C₁-C₆)-Alkyl-Phenyl, (C₂-C₆)-Alkenyl-Phenyl, wobei der Phenylring mit F, Cl, Br, OR13 oder R13 substituiert sein kann;
R4 (C₁-C₆)-Alkyl, (C₁-C₆)-Alkylen-D;
R13 (C₁-C₆)-Alkyl, Phenyl;
sowie deren physiologisch verträgliche Salze, zur Herstellung eines Medikaments zur Blutzuckersenkung.

2. Verwendung der Verbindungen der Formel I, 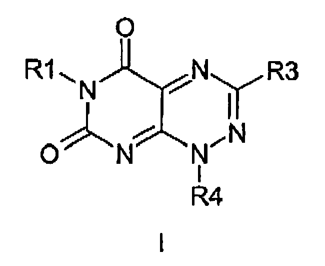 worin bedeuten
R1 (C₁-C₆)-Alkyl;
R3 (C₁-C₆)-Alkyl-Phenyl, (C₂-C₆)-Alkenyl-Phenyl, wobei der Phenylring mit F, Cl, Br, OR13 oder R13 substituiert sein kann;
R4 (C₁-C₆)-Alkyl, (C₁-C₆)-Alkylen-D;
R13 (C₁-C₆)-Alkyl, Phenyl;
sowie deren physiologisch verträgliche Salze, zur Herstellung eines Medikamentes zur Behandlung des Typ II Diabetes.

3. Verwendung der Verbindungen der Formel I, 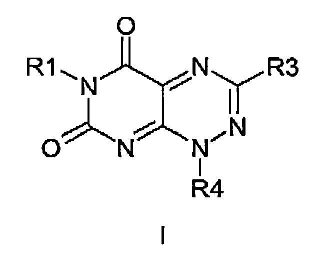 worin bedeuten
R1 (C₁-C₆)-Alkyl;
R3 (C₁-C₆)-Alkyl-Phenyl, (C₂-C₆)-Alkenyl-Phenyl, wobei der Phenylring mit F, Cl, Br, OR13 oder R13 substituiert sein kann;
R4 (C₁-C₈)-Alkyl, (C₁-C₆)-Alkylen-D;
R13 (C₁-C₆)-Alkyl, Phenyl;
sowie deren physiologisch verträgliche Salze, zur Herstellung eines Medikaments zur Behandlung von Lipid- und Kohlenhydratstoffwechselstörungen.

4. Verwendung der Verbindungen der Formel I, 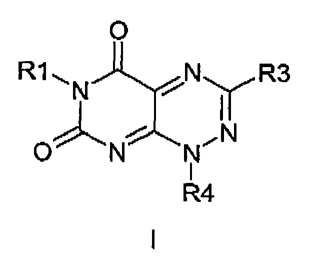 worin bedeuten
R1 (C₁-C₆)-Alkyl;
R3 (C₁-C₆)-Alkyl-Phenyl, (C₂-C₆)-Alkenyl-Phenyl, wobei der Phenylring mit F, Cl, Br, OR13 oder R13 substituiert sein kann;
R4 (C₁-C₆)-Alkyl, (C₁-C₆)-Alkylen-D;
R13 (C₁-C₆)-Alkyl, Phenyl;
sowie deren physiologisch verträgliche Salze, zur Herstellung eines Medikaments zur Behandlung arteriosklerotischer Erscheinungen.

5. Verwendung der Verbindungen der Formel I, 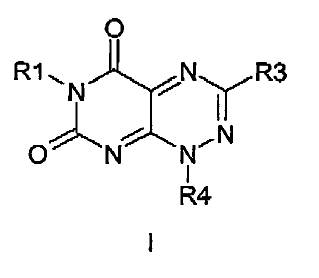 worin bedeuten
R1 (C₁-C₆)-Alkyl;
R3 (C₁-C₆)-Alkyl-Phenyl, (C₂-C₆)-Alkenyl-Phenyl, wobei der Phenylring mit F, Cl, Br, OR13 oder R13 substituiert sein kann;
R4 (C₁-C₆)-Alkyl, (C₁-C₆)-Alkylen-D;
R13 (C₁-C₆)-Alkyl, Phenyl;
sowie deren physiologisch verträgliche Salze, zur Herstellung eines Medikaments zur Behandlung von Insulin Resistenz.

6. Verwendung der Verbindungen der Formeln 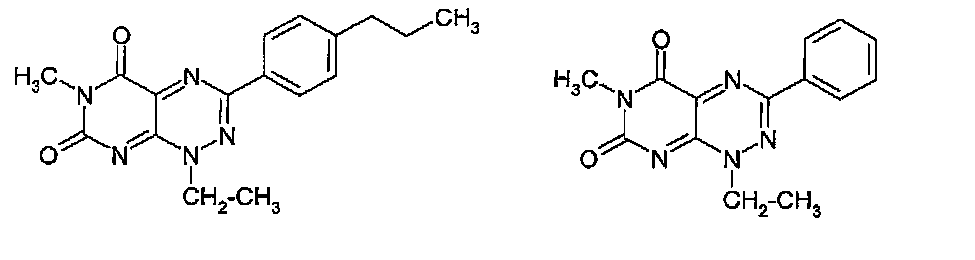 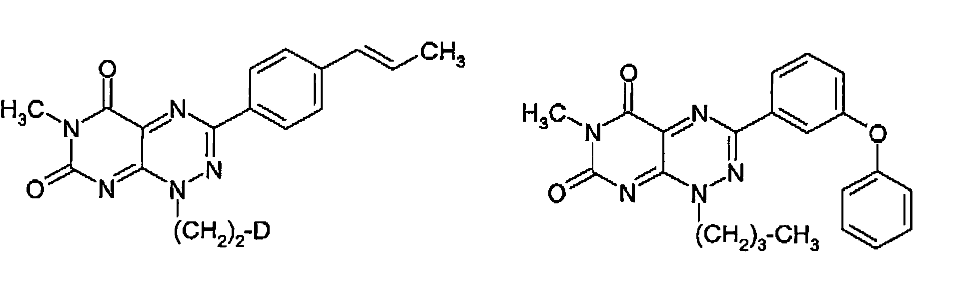 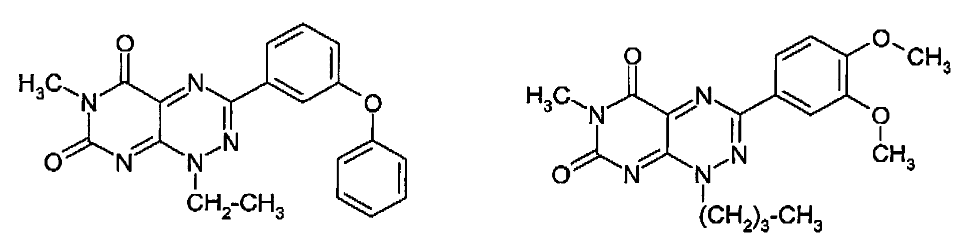 sowie deren physiologisch verträgliche Salze, zur Herstellung eines Medikaments zur Blutzuckersenkung.

7. Verwendung der Verbindungen der Formeln 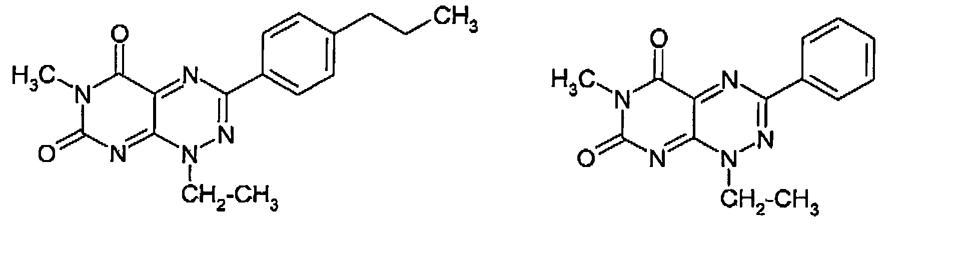 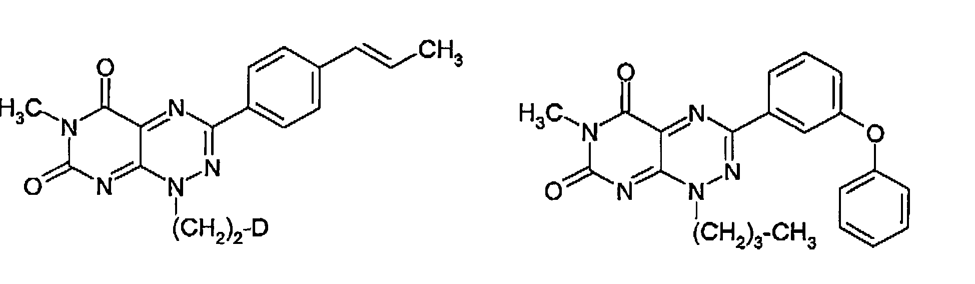 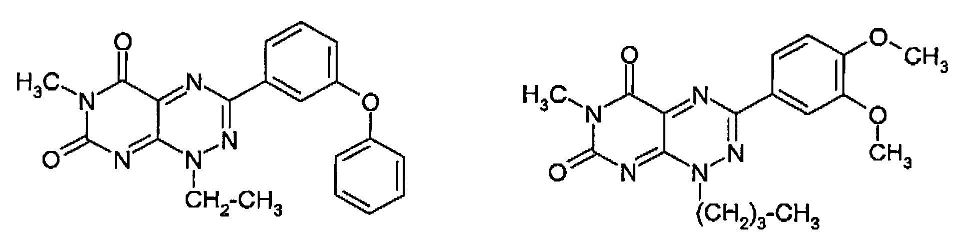 sowie deren physiologisch verträgliche Salze, zur Herstellung eines Medikamentes zur Behandlung des Typ II Diabetes.

8. Verwendung der Verbindungen der Formeln 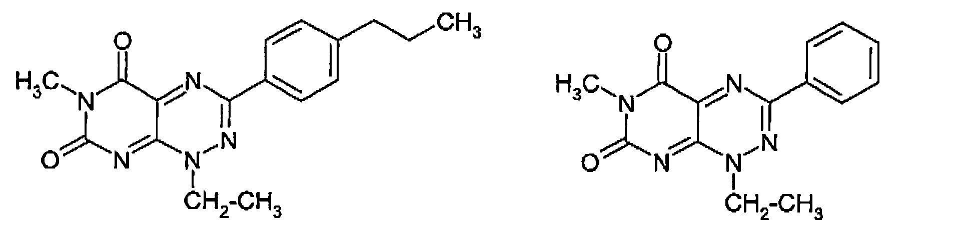 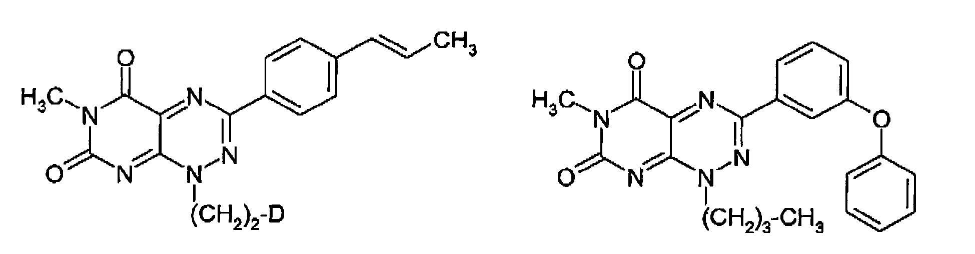 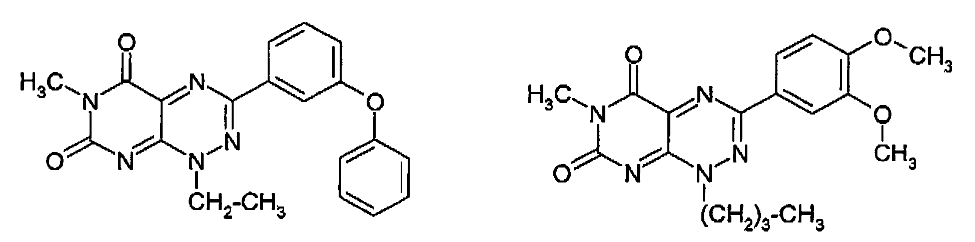 sowie deren physiologisch verträgliche Salze, zur Herstellung eines Medikaments zur Behandlung von Lipid- und Kohlenhydratstoffwechselstörungen.

9. Verwendung der Verbindungen der Formeln 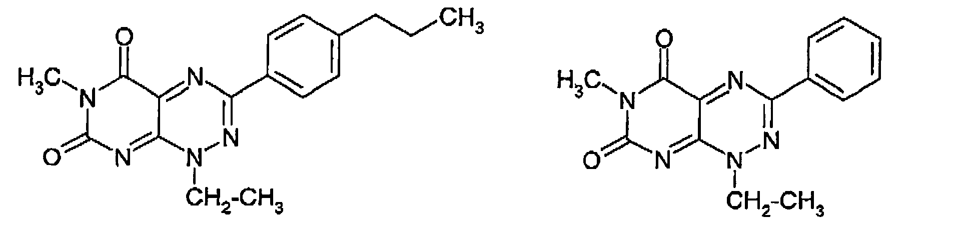 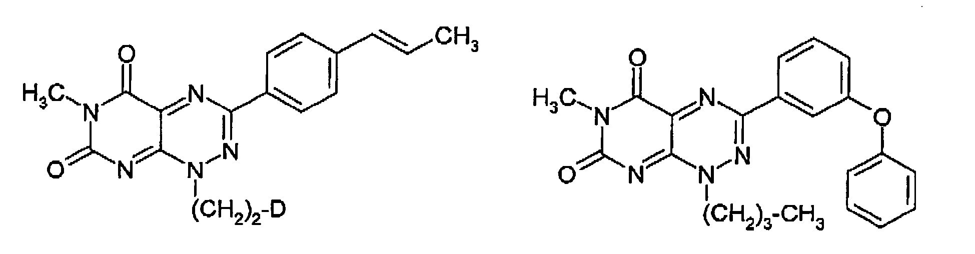 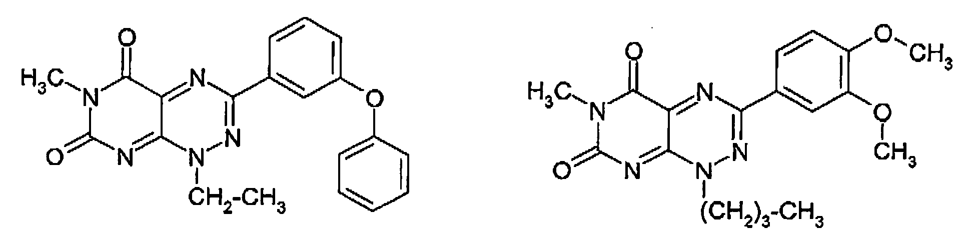 sowie deren physiologisch verträgliche Salze, zur Herstellung eines Medikaments zur Behandlung arteriosklerotischer Erscheinungen.

10. Verwendung der Verbindungen der Formeln 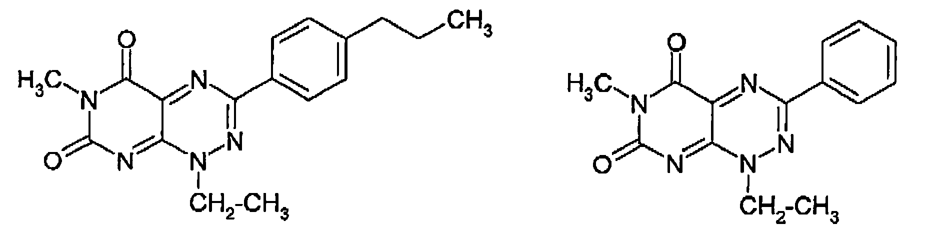 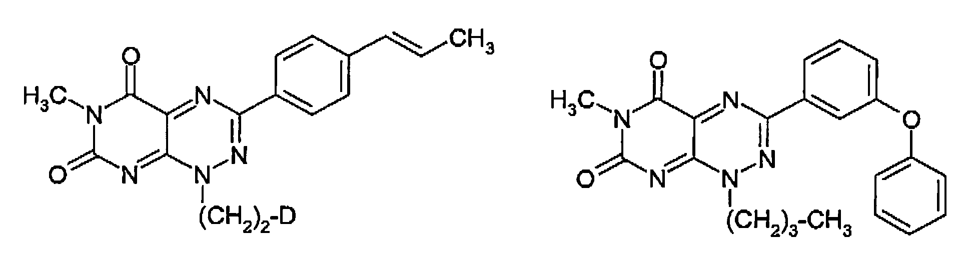 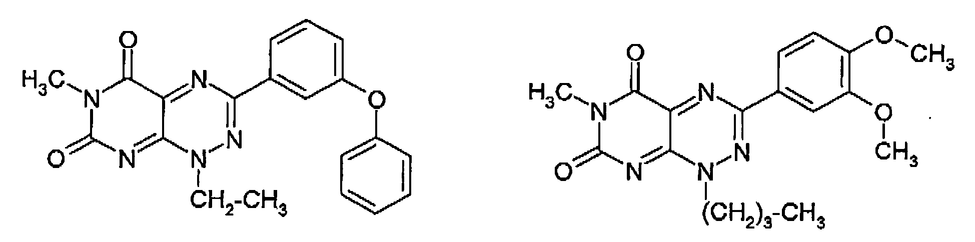 sowie deren physiologisch verträgliche Salze, zur Herstellung eines Medikaments zur Behandlung von Insulin Resistenz.

## Claims

1. The use of the compounds of the formula I, 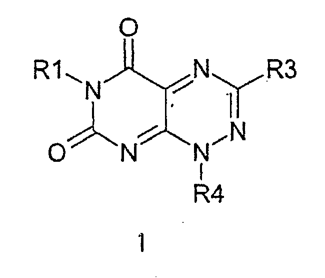 in which
R1 is (C₁-C₆)-alkyl;
R3 is (C₁-C₆)-alkyl-phenyl or (C₂-C₆)-alkenyl-phenyl, where the phenyl ring can be substituted by F, Cl, Br, OR13 or R13;
R4 is (C₁-C₆)-alkyl or (C₁-C₆)-alkylene-D;
R13 is (C₁-C₆)-alkyl or phenyl;
and the physiologically tolerated salts thereof for producing a medicament for lowering blood sugar.

2. The use of the compounds of the formula I, 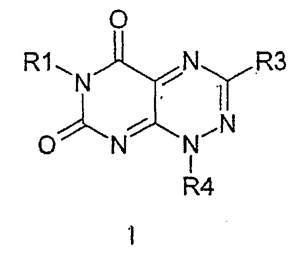 in which
R1 is (C₁-C₆)-alkyl ;
R3 is (C₁-C₆)-alkyl-phenyl or (C₂-C₆)-alkenyl-phenyl, where the phenyl ring can be substituted by F, Cl, Br, OR13 or R13;
R4 is (C₁-C₆)-alkyl or (C₁-C₆)-alkylene-D;
R13 is (C₁-C₆)-alkyl or phenyl;
and the physiologically tolerated salts thereof for producing a medicament for treating type II diabetes.

3. The use of the compounds of the formula I, 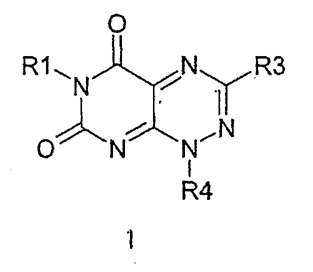 in which
R1 is (C₁-C₆)-alkyl;
R3 is (C₁-C₆)-alkyl-phenyl or (C₂-C₆)-alkenyl-phenyl, where the phenyl ring can be substituted by F, Cl, Br, OR13 or R13;
R4 is (C₁-C₆)-alkyl or (C₁-C₆)-alkylene-D;
R13 is (C₁-C₆)-alkyl or phenyl;
and the physiologically tolerated salts thereof for producing a medicament for treating disturbances of lipid and carbohydrate metabolism.

4. The use of the compounds of the formula I, 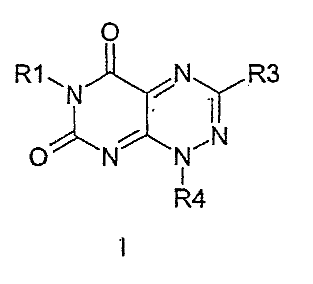 in which
R1 is (C₁-C₆)-alkyl ;
R3 is (C₁-C₆)-alkyl-phenyl or (C₂-C₆)-alkenyl-phenyl, where the phenyl ring can be substituted by F, Cl, Br, OR13 or R13;
R4 is (C₁-C₆)-alkyl or (C₁-C₆)-alkylene-D;
R13 is (C₁-C₆)-alkyl or phenyl;
and the physiologically tolerated salts thereof for producing a medicament for treating arteriosclerotic symptoms.

5. The use of the compounds of the formula I, 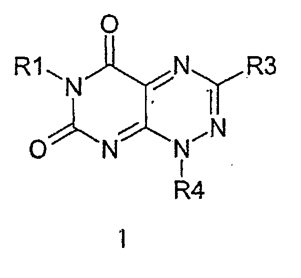 in which
R1 is (C₁-C₆)-alkyl;
R3 is (C₁-C₆)-alkyl-phenyl or (C₂-C₆)-alkenyl-phenyl, where the phenyl ring can be substituted by F, Cl, Br, OR13 or R13;
R4 is (C₁-C₆)-alkyl or (C₁-C₆)-alkylene-D;
R13 is (C₁-C₆)-alkyl or phenyl;
and the physiologically tolerated salts thereof for producing a medicament for treating insulin resistance.

6. The use of the compounds of the formulae 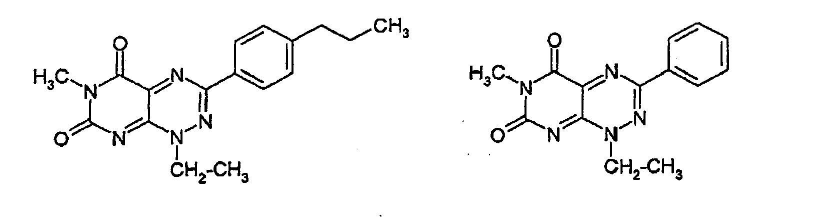 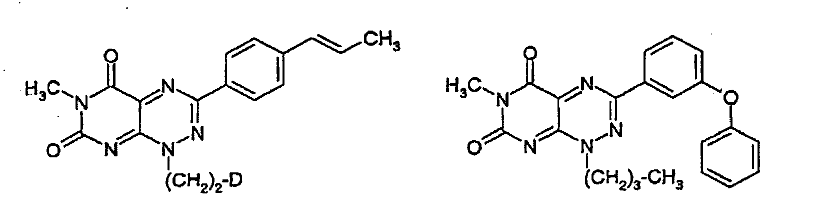 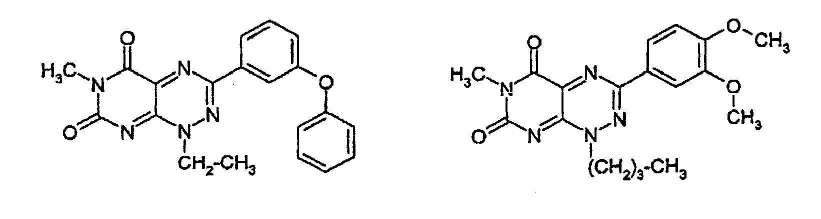 and the physiologically tolerated salts thereof, for producing a medicament for lowering blood sugar.

7. The use of the compounds of the formulae 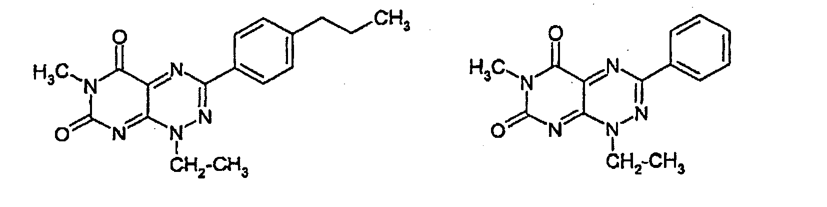 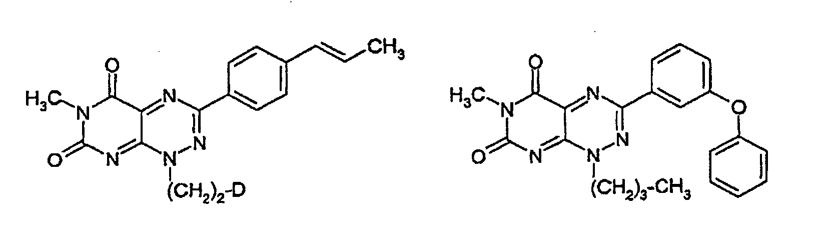 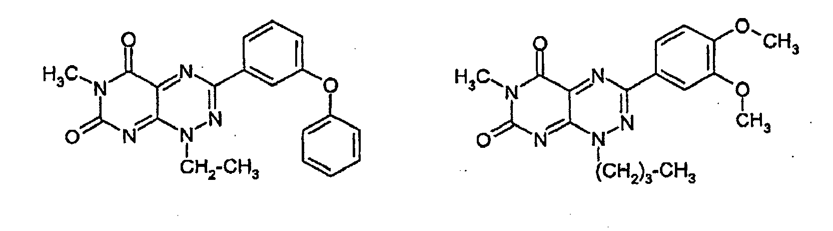 and the physiologically tolerated salts thereof, for producing a medicament for treating type II diabetes.

8. The use of the compounds of the formulae 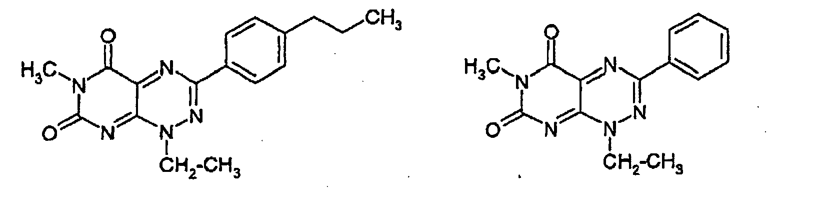 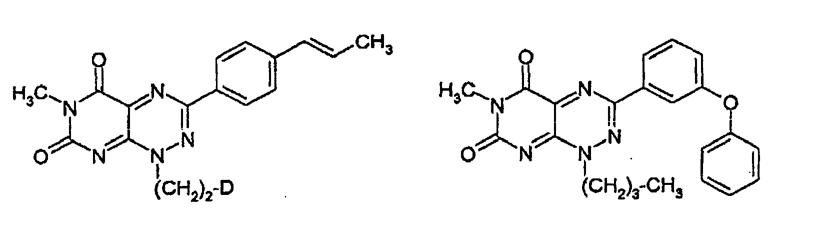 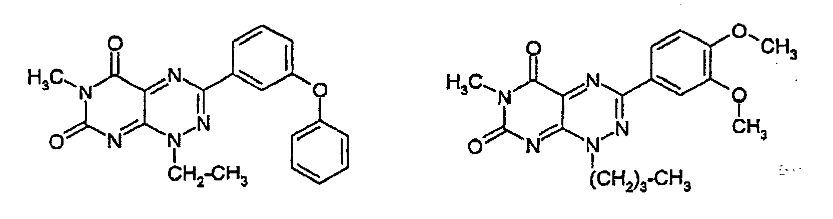 and the physiologically tolerated salts thereof, for producing a medicament for treating disturbances of lipid and carbohydrate metabolism.

9. The use of the compounds of the formulae 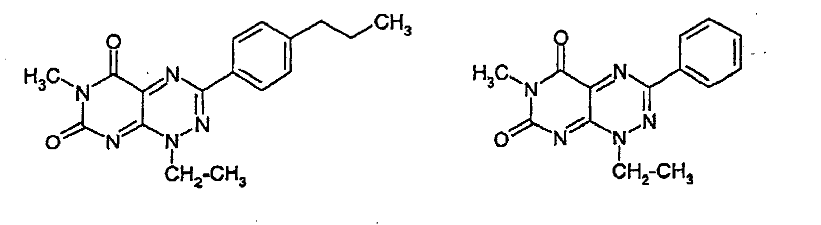 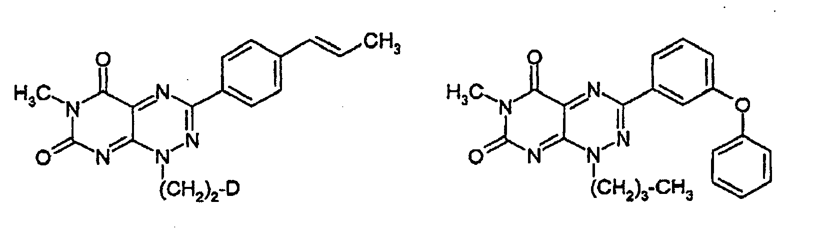 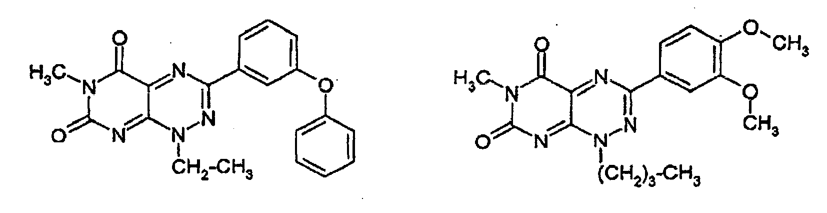 and the physiologically tolerated salts thereof, for producing a medicament for treating arteriosclerotic symptoms.

10. The use of the compounds of the formulae 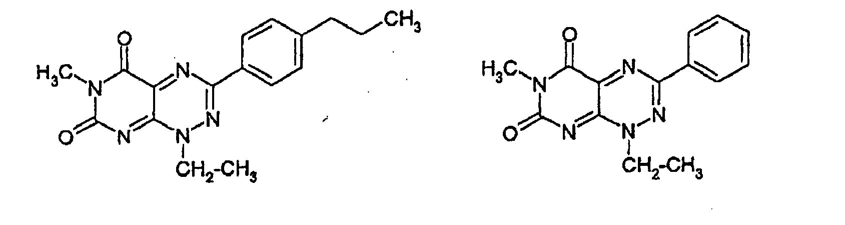 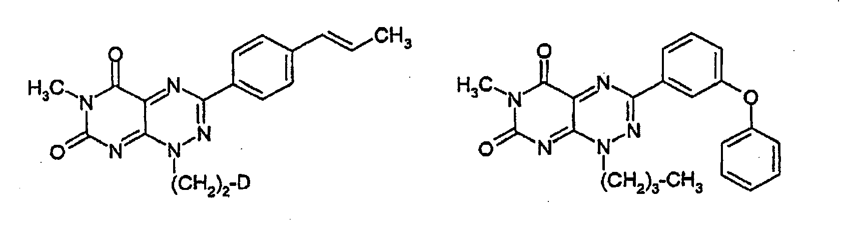 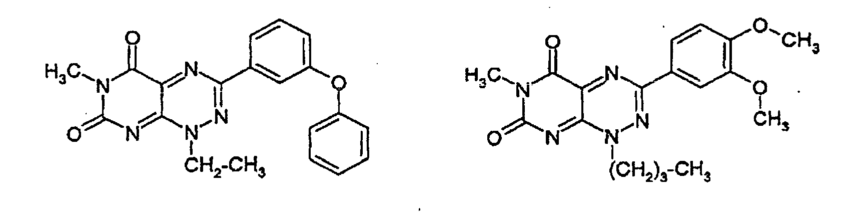 and the physiologically tolerated salts thereof, for producing a medicament for treating insulin resistance.

## Revendications

1. Utilisation des composés de formule I, 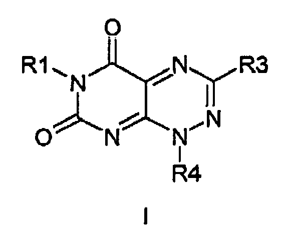 dans laquelle
R1 signifie (C₁-C₆)-alkyle ;
R3 signifie (C₁-C₆)-alkyl-phényle, (C₂-C₆)alcényl-phényle, où le cycle phényle peut être substitué par F, Cl, Br, OR13 ou R13 ;
R4 signifie (C₁-C₆)-alkyle, (C₁-C₆)-alkylène-D ;
R13 signifie (C1-C6)-alkyle, phényle ;
ainsi que leurs sels physiologiquement acceptables pour la préparation d'un médicament destiné à la diminution de la glycémie.

2. Utilisation des composés de formule I, 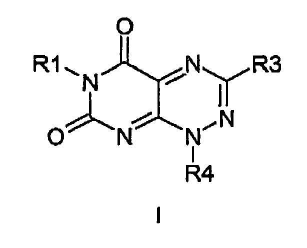 dans laquelle
R1 signifie (C₁-C₆) -alkyle ;
R3 signifie (C₁-C₆) -alkyl-phényle, (C₂ -C₆) alcényl-phényle, où le cycle phényle peut être substitué par F, Cl, Br, OR13 ou R13 ;
R4 signifie (C₁-C₆)-alkyle, (C₁-C₆)-alkylène-D ;
R13 signifie (C₁-C₆)-alkyle, phényle ;
ainsi que leurs sels physiologiquement acceptables pour la préparation d'un médicament destiné au traitement du diabète de type II.

3. Utilisation des composés de formule I, 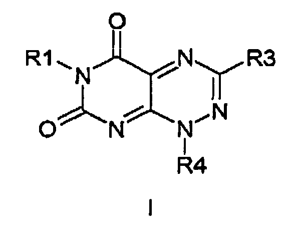 dans laquelle
R1 signifie (C₁-C₆)-alkyle ;
R3 signifie (C₁-C₆) -alkyl-phényle, (C₂-C₆)alcényl-phényle, où le cycle phényle peut être substitué par F, Cl, Br, OR13 ou R13 ;
R4 signifie (C₁-C₆)-alkyle, (C₁-C₆)-alkylène-D ;
R13 signifie (C₁-C₆)-alkyle, phényle ;
ainsi que leurs sels physiologiquement acceptables pour la préparation d'un médicament destiné au traitement de troubles du métabolisme des lipides et des hydrates de carbone.

4. Utilisation des composés de formule I, 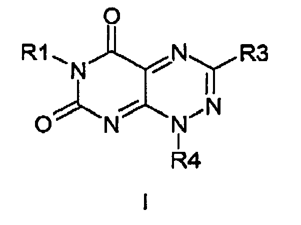 dans laquelle
R1 signifie (C₁-C₆)-alkyle ;
R3 signifie (C₁-C₆)-alkyl-phényle, (C₂-C₆)alcényl-phényle, où le cycle phényle peut être substitué par F, Cl, Br, OR13 ou R13 ;
R4 signifie (C₁-C₆)-alkyle, (C₁-C₆)-alkylène-D ;
R13 signifie (C₁-C₆)-alkyle, phényle ;
ainsi que leurs sels physiologiquement acceptables pour la préparation d'un médicament destiné au traitement de troubles artérioscléreux.

5. Utilisation des composés de formule I, 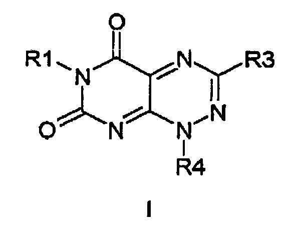 dans laquelle
R1 signifie (C₁-C₆)-alkyle ;
R3 signifie (C₁-C₆)-alkyl-phényle, (C₂-C₆)alcényl-phényle, où le cycle phényle peut être substitué par F, Cl, Br, OR13 ou R13 ;
R4 signifie (C₁-C₆) -alkylène-D ;
R13 signifie (C₁-C₆) -alkyle, phényle ;
ainsi que leurs sels physiologiquement acceptables pour la préparation d'un médicament destiné au traitement de l'insuline.

6. Utilisation des composés de formule 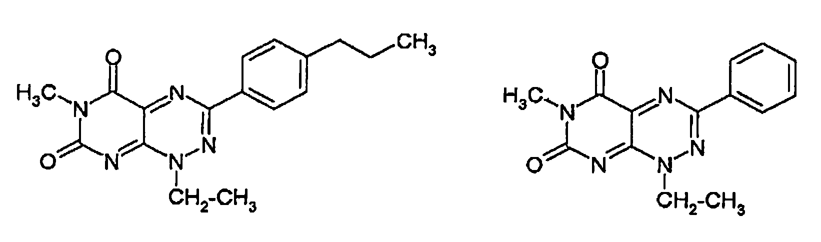 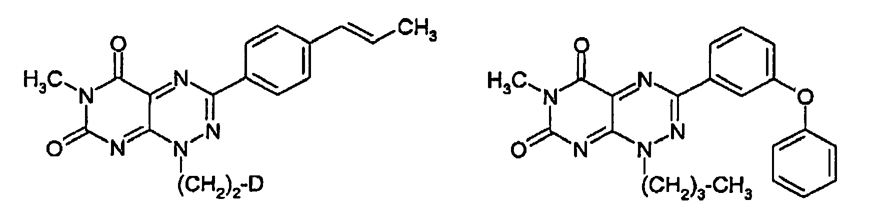 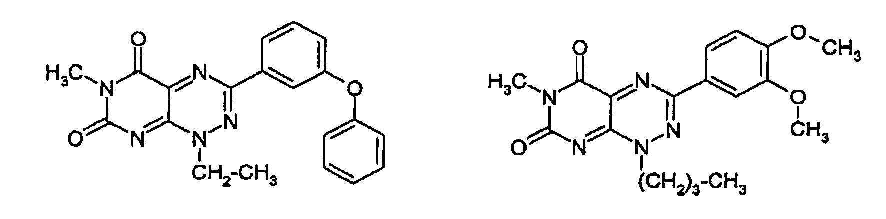 ainsi que leurs sels physiologiquement acceptables pour la préparation d'un médicament destiné à la diminution de la glycémie.

7. Utilisation des composés de formule 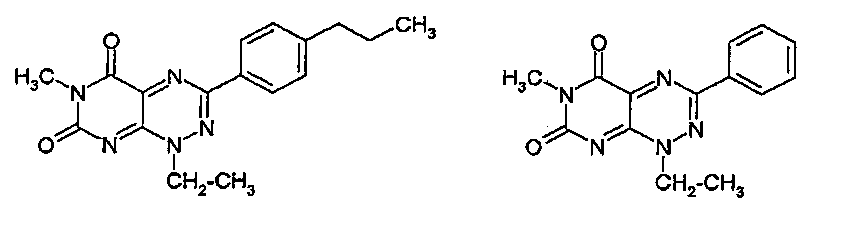 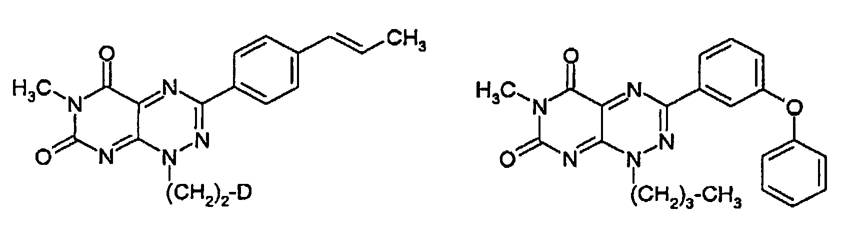 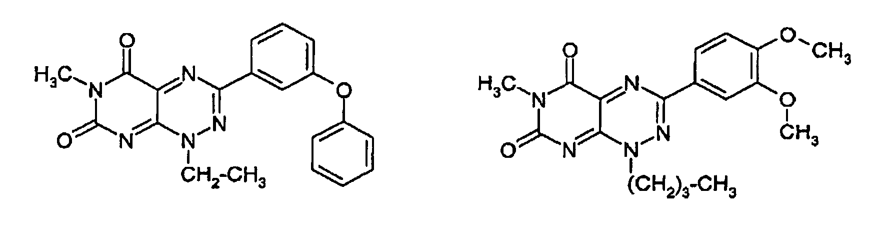 ainsi que leurs sels physiologiquement acceptables pour la préparation d'un médicament destiné au traitement du diabète de type II.

8. Utilisation des composés de formule 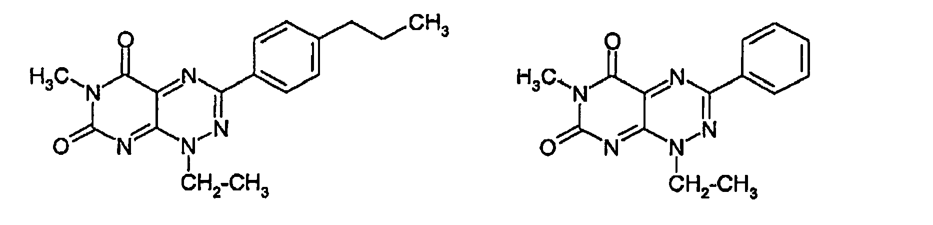 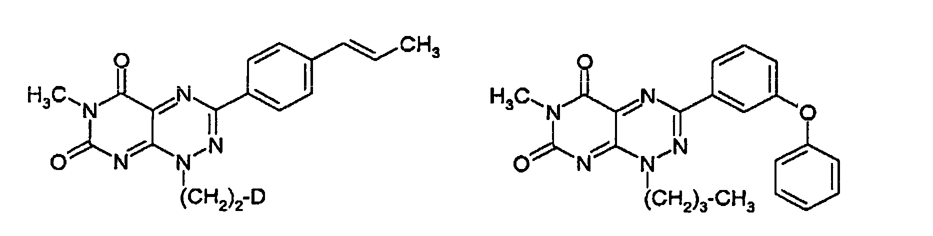 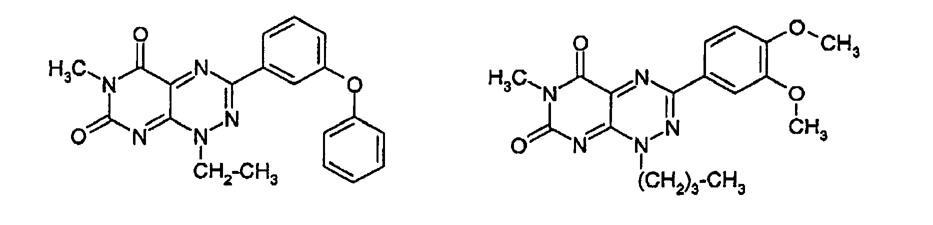 ainsi que leurs sels physiologiquement acceptables pour la préparation d'un médicament destiné au traitement de troubles du métabolisme des lipides et des hydrates de carbone.

9. Utilisation des composés de formule 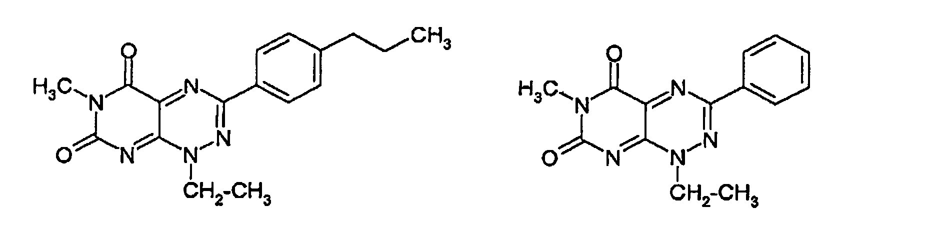 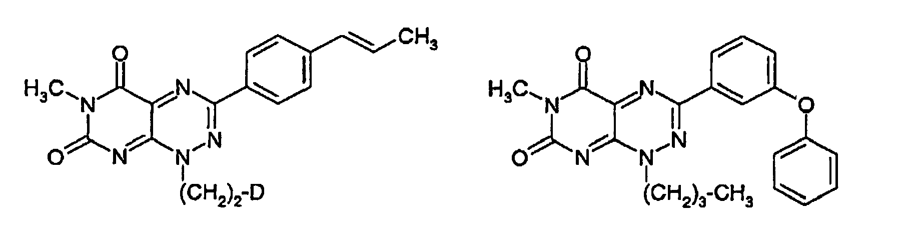 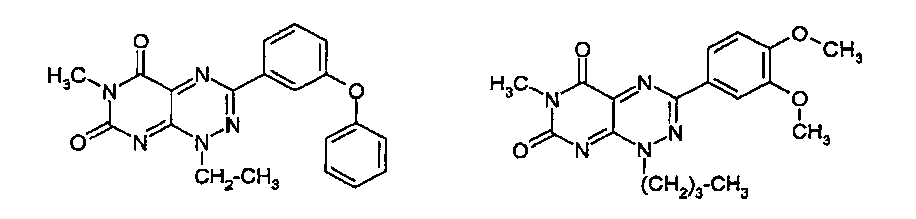 ainsi que leurs sels physiologiquement acceptables pour la préparation d'un médicament destiné au traitement de troubles artérioscléreux.

10. Utilisation des composés de formule 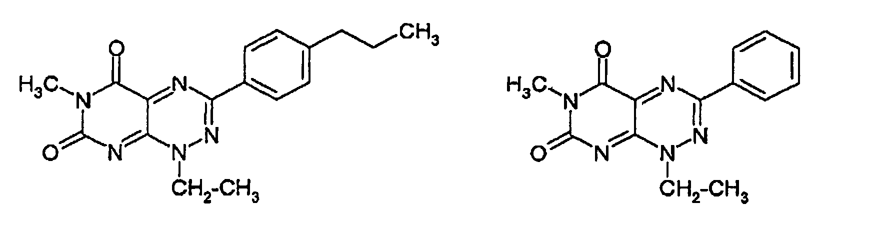 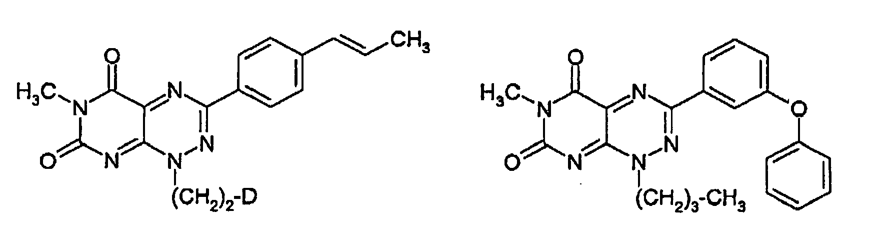 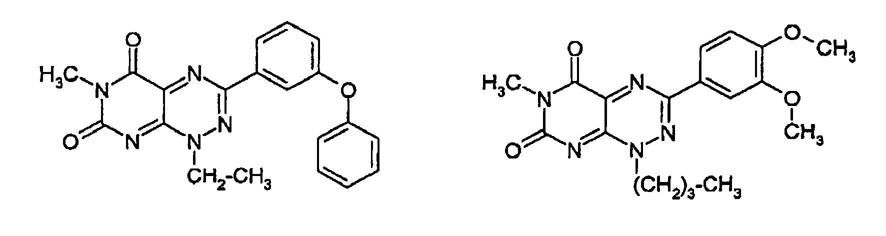 ainsi que leurs sels physiologiquement acceptables pour la préparation d'un médicament destiné au traitement de l'insuline.
